# EUROPÄISCHE PATENTSCHRIFT

(11) **EP 3 474 756 B1**
(45) Veröffentlichungstag und Bekanntmachung des Hinweises auf die Patenterteilung: **25.08.2021**
(21) Anmeldenummer: 17736881.8
(22) Anmeldetag: 14.06.2017
(51) Int. Cl.: A61B 17/12

(54) **IMPLANTAT MIT ABLÖSEMECHANISMUS**
IMPLANT HAVING A DETACHABLE MECHANISM
IMPLANT COMPORTANT UN MÉCANISME DE SÉPARATION

(30) Priorität: 23.06.2016 DE 102016111568
(43) Veröffentlichungstag der Anmeldung: 01.05.2019
(73) Patentinhaber: Phenox GmbH, 44801 Bochum (DE)
(72) Erfinder: HENKES, Hans, 70192 Stuttgart (DE); MONSTADT, Hermann, 44797 Bochum (DE); HANNES, Ralf, 44137 Dortmund (DE)
(74) Vertreter: Schneiders & Behrendt Bochum
(86) Internationale Anmeldenummer: PCT/EP2017/064530
(87) Internationale Veröffentlichungsnummer: WO 2017/220400

(56) Entgegenhaltungen:
- WO-A1-02/00139
- WO-A1-2010/028314
- WO-A2-2005/070308
- US-A1- 2003 055 440
- US-A1- 2012 296 362
- US-A1- 2014 121 752

## Beschreibung

Die Erfindung betrifft ein Implantat zum Einsatz bei der Okkludierung von Aneurysmen in Blutgefäßen im Bereich von Gefäßverzweigungen, insbesondere Bifurkationsaneurysmen, wobei das Implantat in einem expandierten Zustand, in dem es im Blutgefäß implantiert wird, und in einem kontrahierten Zustand vorliegt, in dem es durch das Blutgefäß bewegbar ist, wobei das Implantat einen proximalen Fixierabschnitt, mit dem das Implantat an einer Gefäßwand des Blutgefäßes fixierbar ist, einen distalen Abschnitt, in dem das Implantat im expandierten Zustand gegenüber dem Fixierabschnitt radial erweitert ist und der zur Platzierung im oder vor dem Aneurysma bestimmt ist, und einen Übergangsabschnitt zwischen dem Fixierabschnitt und dem distalen Abschnitt aufweist, wobei der Fixierabschnitt über eine 1. Ablösestelle ablösbar mit einer Einführhilfe verbunden ist und wobei der Übergangsabschnitt eine 2. Ablösestelle aufweist, die eine Abtrennung des distalen Abschnitts ermöglicht. Darüber hinaus betrifft die Erfindung ein Verfahren zur Einbringung des Implantats.

Arteriovenöse Fehlbildungen können in einem Patienten zu erheblichen Beeinträchtigungen und Gefährdungen bis hin zum Tode führen. Dies gilt besonders für Aneurysmen, insbesondere dann, wenn sie im zerebralen Bereich auftreten. In der Regel versucht man, derartige Fehlbildungen durch Implantate zu verschließen. Solche Implantate werden zumeist auf endovaskulärem Weg mit Hilfe von Kathetern gesetzt.

Insbesondere bei zerebralen Aneurysmen hat sich die Implantierung von Platinspiralen bewährt, die das Aneurysma mehr oder weniger vollständig ausfüllen, den Bluteinstrom weitgehend blockieren und dazu führen, dass sich ein lokaler Thrombus ausbildet, der das Aneurysma ausfüllt und letztlich verschließt. Diese Behandlungsmethode ist allerdings nur bei Aneurysmen geeignet, die über einen relativ engen Zugang zum Gefäßsystem verfügen, sogenannten Beerenaneurysmen. Bei Aussackungen von Blutgefäßen, die über einen weiten Zugang zum Gefäß verfügen, drohen die implantierten Spiralen wieder ausgeschwemmt zu werden. Diese können in andere Bereiche des Gefäßsystems gelangen und dort Schäden herbeiführen.

In solchen Fällen wurde bereits vorgeschlagen, eine Art Stent zu setzen, der die Öffnung des Aneurysmas "vergittert" und dadurch die Ausschwemmung der Okklusionsspiralen verhindert. Derartige Stents, die über eine relativ weitmaschige Wandung verfügen, werden bereits bei einigen Formen von Aneurysmen eingesetzt.

Gefäßverzweigungen, insbesondere Gefäßbifurkationen, sind ein relativ häufiges Phänomen. Das durch eine Arterie im Bereich einer Gabelung auf die Stirnwand aufprallende Blut führt - im Fall einer Schwächung der Gefäßwandung - schnell zu einer Aussackung, die sich dann rasch erweitert. Solche Bifurkationsaneurysmen haben häufig einen weiten Hals, der eine Therapie nur mit Okklusionsspiralen unmöglich macht.

Vaskuläre Implantate, die geeignet sind, im Bereich einer Gefäßverzweigung eine "Vergitterung" des Aneurysmaeingangs herbeizuführen, werden beispielsweise in den internationalen Patentanmeldungen WO 2012/113554 A1 oder WO 2014/029835 A1 offenbart. Mit nach dem Setzen des Implantats eingebrachten Okklusionsspiralen kann dann das Aneurysma stillgelegt werden. Möglich ist auch, dass das Implantat selber das Aneurysma in ausreichendem Maße vom Blutstrom abkoppelt. Hierzu kann beispielsweise das Implantat eine Membran aufweisen, die im Bereich des Aneurysmahalses oder vor dem Aneurysmahals platziert wird. Ggf. kann auch allein mit Filamenten, typischerweise Drähten geringen Durchmessers, der Blutstrom zum Aneurysma so weit reduziert werden, dass auf die zusätzliche Einbringung von Okklusionsspiralen oder anderen Okklusionsmitteln in das Aneurysma verzichtet werden kann.
Aus dem Stand der Technik bekannte Implantate weisen einen proximalen Abschnitt auf, der der Fixierung des Implantats im Trägergefäß dient und im Wesentlichen die Form eines herkömmlichen Stents aufweist. Am distalen Ende des Implantats ist ein distaler Abschnitt vorgesehen, der im oder vor dem Aneurysma platziert werden soll, um das Aneurysma vom Blutfluss abzuschneiden und/oder in das Aneurysma eingebrachte Okklusionsmittel daran zu hindern, aus dem Aneurysma in das Blutgefäß auszutreten. Zwischen dem proximalen und dem distalen Abschnitt kann ein Zwischenabschnitt vorgesehen sein, der beispielsweise über eine relativ geringe Dichte der Filamente verfügt, um den Blutfluss in abzweigende Blutgefäße nicht oder nur geringfügig zu behindern.
Der schaftartig aufgebaute, proximale Abschnitt, der der Fixierung des Implantats im Trägergefäß dient, hat sich in vielen Fällen als wertvoll bei der Platzierung des distalen Abschnitts vor dem Aneurysma, im Bereich des Aneurysmahalses bzw. im Aneurysma selbst herausgestellt. Es treten jedoch auch Fälle auf, in denen sich eine zusätzliche Fixierung des distalen Abschnitts durch einen schaftartigen Fixierabschnitt als nicht erforderlich erweist, da der distale Abschnitt selbst sich bereits hinreichend gut im Eintrittsbereich des Aneurysmas verankern lässt. In solchen Fällen ist das Verbleiben des Fixierabschnitts im Blutgefäßsystem eigentlich unnötig. Grundsätzlich gilt, dass mit jedem zusätzlichen Implantat die Gefahr des Auftretens weiterer Komplikationen verbunden sein kann. Insbesondere kann, je nach Gefäßgeometrie, ein zu langer Fixierabschnitt zu Komplikationen bei der Implantation führen. Die US2014/121752 A1 weist einen vom proximalen Fixierabschnitt teilweise trennbaren distalen Abschnitt auf. Der proximale Fixierabschnitt ist von der Einführhilfe ablösbar.

Ausgehend von Implantaten, wie sie beispielsweise in der US2014/121752 A1 beschrieben werden, stellt sich daher die Aufgabe, ein entsprechendes Implantat zur Verfügung zu stellen, dass für den Arzt mehr Variabilität in der Handhabung mit sich bringt und insbesondere auch eine Platzierung lediglich des distalen Abschnitts vor dem Aneurysma, im Aneurysmahals oder im Aneurysma erlaubt.

Diese Aufgabe wird erfindungsgemäß gelöst durch ein gemäß Anspruch 1 definiertes Implantat zum Einsatz bei der Okkludierung von Aneurysmen in Blutgefäßen im Bereich von Gefäßverzweigungen, insbesondere Bifurkationsaneurysmen, wobei das Implantat in einem expandierten Zustand, in dem es im Blutgefäß implantiert wird, und in einem kontrahierten Zustand vorliegt, in dem es durch das Blutgefäß bewegbar ist, wobei das Implantat einen proximalen Fixierabschnitt, mit dem das Implantat an einer Gefäßwand des Blutgefäßes fixierbar ist, einen distalen Abschnitt, in dem das Implantat im expandierten Zustand gegenüber dem Fixierabschnitt radial erweitert ist und der zur Platzierung im oder vor dem Aneurysma bestimmt ist, und einen Übergangsabschnitt zwischen dem Fixierabschnitt und dem distalen Abschnitt aufweist, wobei der Fixierabschnitt über eine 1. Ablösestelle ablösbar mit einer Einführhilfe verbunden ist und wobei der Übergangsabschnitt eine 2. Ablösestelle aufweist, die eine Abtrennung des distalen Abschnitts ermöglicht.
Durch die Schaffung einer 2. Ablösestelle im Übergangsabschnitt zwischen Fixierabschnitt und distalem Abschnitt erhält der behandelnde Arzt zusätzliche Möglichkeiten. Bei der gewöhnlichen Einbringung des Implantats kann er eine Abtrennung an der 1. Ablösestelle wählen, so dass der schaftartige Fixierabschnitt im Blutgefäßsystem verbleibt und dort für eine Verankerung sorgt. Der distale Abschnitt des Implantats wird vor dem Aneurysma oder im Aneurysma platziert und durch den Fixierabschnitt in der entsprechenden Position gehalten.
Wenn der behandelnde Arzt jedoch feststellt, dass der distale Abschnitt allein bereits ausreichend im Aneurysmahals/Aneurysma oder auch vor dem Aneurysma fixiert ist und keine Gefahr droht, dass der distale Abschnitt durch den Blutstrom fortbewegt wird, kann sich der Arzt auch dazu entscheiden, eine Ablösung an der 2. Ablösestelle zu wählen. In diesem Fall verbleibt nur der distale Abschnitt im Blutgefäßsystem; die Einführhilfe wird zusammen mit dem Fixierabschnitt zurückgezogen und aus dem Blutgefäß entfernt. Ein ausschließliches Belassen des distalen Abschnitts kann auch dann sinnvoll sein, wenn erkennbar ist, dass die Platzierung des länglichen Fixierabschnitts mit Schwierigkeiten verbunden ist, beispielsweise wegen starker Windungen der Blutgefäße oder weiteren Abzweigungen proximal des Aneurysmas.

Bevorzugt handelt es sich bei dem Implantat um ein selbstexpandierendes Implantat aus Formgedächtnismaterialien, sodass die Expansion und die Anlegung an die Gefäßinnenwand selbständig erfolgt. Alternativ kann das Implantat, insbesondere der stentähnliche Fixierabschnitt auch mit Hilfe eines Ballons, auf den der Fixierabschnitt aufgecrimpt ist, oder anderen mechanischen Verfahren aufgeweitet werden.

Der Fixierabschnitt weist eine stentähnliche Röhrenstruktur auf. Diese kann entweder lasergeschnitten sein, so dass sich eine Oberfläche aus Stegen ergibt, zwischen denen Öffnungen vorliegen. Zusätzlich ist es sinnvoll, die Stege einer Elektropolitur zu unterziehen, um diese glatter und abgerundeter und damit weniger traumatisch werden zu lassen. Darüber hinaus sinkt die Gefahr der Anhaftung von Keimen oder sonstigen Verunreinigungen.

Alternativ kann die Röhrenstruktur auch aus einem Drahtgeflecht aufgebaut werden, welches eine Maschenstruktur ausbildet. Die Drähte verlaufen hierbei typischerweise helixförmig entlang der Längsachse, wobei gegenläufig verlaufende Drähte an den Kreuzungspunkten über- und untereinander her verlaufen, sodass sich wabenförmige Öffnungen zwischen den Drähten ausbilden. Die Gesamtzahl der Drähte beträgt bevorzugt 3 bis 64. Bei den Drähten, die die Maschenstruktur ausbilden, kann es sich um einzelne Drähte aus Metall handeln, möglich ist aber auch das Vorsehen von Litzen, d. h. mehreren Drähten geringen Durchmessers, die zusammen ein Filament bilden und vorzugsweise miteinander verdrillt sind.

Der Fixierabschnitt des Implantats hat im expandierten Zustand typischerweise eine Länge von 5 bis 40 mm, bevorzugt 10 bis 20 mm. Der Durchmesser beträgt im expandierten Zustand meist 1 bis 10 mm, insbesondere 3 bis 6 mm. Ein solcher Durchmesser ist für neurovaskuläre Anwendungen bevorzugt. Die Länge des Übergangsabschnitts beträgt bei expandiertem Implantat zumeist 1 bis 15 mm, bevorzugt 5 bis 12 mm.

Die sich in der Röhrenstruktur des Fixierabschnitts ergebenden Öffnungen sollten rundum geschlossen, d. h. ohne Unterbrechungen von Stegen bzw. Drähten umgeben sein (sog. "Closed-Cell-Design"). Auf diese Weise wird das Befördern des Fixierabschnitts in den Mikrokatheter durch Aufschub desselben erleichtert. Dies ist dann von Bedeutung, wenn der Arzt sich entscheidet, an der 2. Ablösestelle eine Ablösung vorzunehmen und den Fixierabschnitt durch Zurückziehen aus dem Blutgefäß zu entfernen.
Insoweit handelt es sich bei dem Implantat weitgehend um einen Stent oder ein stentähnliches Gebilde, das sich durch die besondere Art seines Einsatzes und Aufbaus auszeichnet. Die Ähnlichkeit mit einem Stent gilt insbesondere für den Fixierabschnitt, während der distale Abschnitt radial nach außen erweitert ist und beispielsweise nach außen weisende Bögen aufweisen kann.
Die Drähte oder Stege sind insbesondere aus Metall gefertigt. Besonders bevorzugt ist die Verwendung von Formgedächtnismetallen wie Nickel-Titan-Legierungen, die auch unter dem Namen Nitinol bekannt sind. Auch ternäre Nickel-Titan-Legierungen können zum Einsatz kommen. Ebenso möglich ist die Verwendung anderer Legierungen, Polymere oder sonstiger Materialien mit Formgedächtniseigenschaften oder herkömmlicher Stentmaterialien wie medizinischer Stahl oder Kobalt-Chrom-Legierungen. Die Verwendung von Materialien mit Formgedächtniseigenschaften ist insofern von Vorteil, als hierdurch sichergestellt wird, dass das Implantat nach Freisetzung selbständig seine expandierte Form annimmt. Neben der Verwendung von Metall können die Stege oder Drähte auch aus einem Polymermaterial gefertigt sein; insofern werden auch Filamente aus einem Polymermaterial als Drähte im Sinne der Erfindung angesehen.
Die Stege/Drähte können einen runden, ovalen, quadratischen oder rechteckigen Querschnitt aufweisen. Möglich ist auch die Verwendung von flachen Stegen/Drähten in Form dünner Streifen, insbesondere Metallstreifen. Im Falle eines quadratischen oder rechteckigen Querschnitts ist eine Abrundung der Kanten von Vorteil.

Für die 1. und die 2. Ablösestelle werden erfindungsgemäß unterschiedliche Ablösemechanismen gewählt. In Frage kommen insbesondere mechanische, elektrolytische oder thermische Ablösemechanismen. Derartige Ablösemechanismen sind grundsätzlich aus dem Stand der Technik bekannt.

Unter einem mechanischen Ablösemechanismus werden solche Mechanismen verstanden, bei denen ein Abschnitt mit mechanischen Mitteln mit einem anderen Abschnitt des Implantats verbunden ist, insbesondere über einen Formschluss. Es muss jedoch möglich sein, von außen Einfluss zu nehmen auf die Verbindung, sodass eine Ablösung und Freisetzung des distal gelegenen Teils des Implantats erfolgt. Z. B. können Verbindungselemente an einem Abschnitt vorgesehen sein, die mit einem entsprechend angepassten Halteelement des benachbarten Abschnitts zusammenwirken. Eine Möglichkeit besteht darin, dass Verbindungselemente und Halteelement so aufeinander abgestimmt sind, dass sie formschlüssig ineinandergreifen und auf diese Weise eine Verbindung herbeiführen. Der Arzt kann jedoch durch bestimmte Maßnahmen erreichen, dass sich der Formschluss auflöst und damit eine Ablösung erfolgt. Beispielsweise können Verbindungselemente und Halteelement von einer Umhüllung umgeben sein, die eine Lösung der Verbindungselemente vom Halteelement verhindert. Wird jedoch diese Umhüllung entfernt, insbesondere in Richtung proximal zurückgezogen, können sich die Verbindungselemente vom Halteelement lösen und der entsprechende Abschnitt des Implantats wird freigesetzt. Das Zusammenwirken zwischen Verbindungs- und Halteelement kann ähnlich dem Schlüssel-Schloss-Prinzip erfolgen. Bei der Umhüllung kann es sich z. B. um einen Schlauch aus Kunststoff, eine Hülse aus Kunststoff oder Metall, eine Spiralwendel aus Metall oder auch um Kombinationen hieraus handeln. Wenn dieser Ablösemechanismus für die 1. Ablösestelle vorgesehen wird, ist eine Fixierung der Umhüllung an der Einführhilfe gegen unbeabsichtigtes Verschieben mit einer Klemmvorrichtung möglich, beispielsweise mit Hilfe eines Torquers. Die Umhüllung muss in diesem Fall nicht die gesamte Einführhilfe abdecken, ausreichend ist eine Erstreckung der Umhüllung über das Halteelement und den distalen Teil der Einführhilfe.

Denkbar ist auch, den für die Zufuhr des Implantats vorgesehenen Mikrokatheter für die Kontrolle der Freisetzung zu verwenden. In diesem Fall erfolgt eine Ablösung des Fixierabschnitts von der Einführhilfe an der 1. Ablösestelle dann, wenn der Mikrokatheter über die 1. Ablösestelle hinaus nach proximal zurückgezogen wird. In diesem Fall muss allerdings darauf geachtet werden, dass der Mikrokatheter erst dann über die 1. Ablösestelle hinaus nach proximal bewegt wird, wenn feststeht, dass eine Ablösung an dieser Position gewünscht ist.

Die Verbindungselemente können beispielsweise kugelförmig ausgebildet sein, wobei die Kugeln von entsprechenden Ausnehmungen im Halteelement umfasst sind, solange keine Ablösung erfolgt. Sobald ein äußerer Zwang, der Verbindungselemente und Halteelement zusammenhält, aufgehoben wird, bewegen sich die kugelförmigen Verbindungselemente aus den Ausnehmungen heraus und eine Freisetzung erfolgt. Anschließend können die proximal der Ablösestelle gelegenen Bereiche des Implantats, im Falle der 1. Ablösestelle die Einführhilfe, im Falle der 2. Ablösestelle die Einführhilfe sowie der Fixierabschnitt, zurückgezogen werden. Selbstverständlich müssen die Verbindungselemente nicht kugelförmig sein, andere Arten geometrischer Formen, insbesondere Verdickungen, die formschlüssig von einem Halteelement erfasst werden können, sind ebenso geeignet. Dabei muss nicht für jedes Verbindungselement eine einzelne Ausnehmung vorhanden sein, möglich ist auch das Vorsehen einer umlaufenden Nut als Ausnehmung, in der die Verbindungselemente gehalten werden. Es ist sowohl möglich, dass die Verbindungselemente am weiter distal gelegenen Abschnitt und das Halteelement am proximal angrenzenden Abschnitt vorliegen als auch eine umgekehrte Anordnung, bei der z. B. Verbindungselemente der Einführhilfe vom Halteelement am Fixierabschnitt formschlüssig umfasst sind.

Die elektrolytische Ablösung von Implantaten ist aus dem Stand der Technik hinlänglich bekannt, beispielsweise für Okklusionscoils zum Verschließen von Aneurysmen. Entsprechende Ablösestellen sind z. B. in der WO 2011/147567 A1 beschrieben. Das Prinzip basiert darauf, dass bei Anlegen einer Spannung eine hierfür vorgesehene Ablösestelle aus einem geeigneten Material, insbesondere Metall, in der Regel durch anodische Oxidation eine zumindest so weit gehende Auflösung erfährt, dass die distal der entsprechenden Ablösestelle gelegenen Bereiche des Implantats freigesetzt werden. Die Ablösestelle kann beispielsweise aus Edelstahl, Magnesium, Magnesiumlegierungen oder einer Cobalt-Chrom-Legierung gefertigt sein.

Die Auflösung der Ablösestelle erfolgt durch Anlegen einer elektrischen Spannung. Dabei kann es sich sowohl um Wechselstrom als auch um Gleichstrom handeln, wobei eine geringe Stromstärke (< 3 mA) ausreicht. Die Ablösestelle stellt dabei in der Regel die Anode dar, an der die Oxidation und Auflösung des Metalls stattfindet.

Die elektrolytische Ablösung erfolgt, indem mit Hilfe einer Spannungsquelle eine elektrische Spannung an die entsprechende Ablösestelle angelegt wird. Diese dient dabei wie erwähnt zumeist als Anode, während die Kathode z. B. auf der Körperoberfläche positioniert werden kann. Selbstverständlich muss die Ablösestelle entsprechend elektrisch leitend, insbesondere über die Einführhilfe, mit der Spannungsquelle verbunden sein. Die Einführhilfe muss in diesem Fall auch selbst elektrisch leitfähig ausgebildet sein. Da der sich einstellende Korrosionsstrom von der Fläche der Kathode gesteuert wird, sollte die Fläche der Kathode deutlich größer gewählt werden als die Fläche der Anode. In gewissem Maße lässt sich die Auflösungsgeschwindigkeit der Ablösestelle durch Einstellung der Kathodenfläche im Verhältnis zur Anodenfläche steuern. Die Erfindung betrifft entsprechend auch eine Vorrichtung, die eine Spannungsquelle sowie ggf. eine auf der Körperoberfläche platzierbare Elektrode umfasst.

Es ist auch möglich, die zweite Elektrode nicht auf der Körperoberfläche zu positionieren, sondern beide Pole direkt am Implantat anzulegen, wodurch die Auflösung der Ablösestelle weiter beschleunigt wird.

Typischerweise weist eine elektrolytische Ablösestelle eine Länge von 0,05 bis 0,5 mm, insbesondere ca. 0,2 mm, und einen Durchmesser von 0,04 bis 0,5 mm, insbesondere ca. 0,1 mm, auf.

Um eine ausreichende Konzentration des Stroms auf eine Ablösestelle herbeizuführen, ist es sinnvoll, benachbarte Bereiche mit einer elektrisch isolierenden Beschichtung zu versehen. Wenn beispielsweise die 2. Ablösestelle elektrolytisch ablösbar ausgebildet ist, ist es zweckmäßig, das Implantat proximal der 2. Ablösestelle ganz oder teilweise mit einer elektrisch isolierenden Beschichtung zu versehen. Insbesondere ist eine Isolierung zwischen der 1. Ablösestelle und der 2. Ablösestelle sinnvoll. Als Beschichtung kann beispielsweise Parylen verwendet werden.

Es ist auch möglich, Ablösestellen vorzusehen, bei denen die Ablösung auf elektrolytischem und mechanischem Weg kombiniert wird. Hierbei wird zwischen Verbindungselementen und Halteelement eine mechanische Verbindung, insbesondere über Formschluss hergestellt, die solange besteht, bis ein die mechanische Verbindung aufrecht erhaltendes Element elektrolytisch korrodiert wird. Gemäß einer Variante befinden sich an einem Abschnitt des Implantats Verdickungen, die vom Halteelement formschlüssig gehalten werden, wobei ein Abschnitt des Halteelements elektrolytisch korrodierbar ausgebildet ist, so dass nach elektrolytischer Auflösung des Abschnitts eine Ablösung erfolgt. Beispielsweise kann die Ablösung des Implantats an der 1. Ablösestelle durch die elektrolytische Korrosion eines Abschnitts des Halteelements an der Einführhilfe herbeigeführt werden. Der korrodierbare Abschnitt ist dabei so angeordnet, dass er den Austritt der in das Halteelement hineinragenden Verdickungen verhindert. Es kann sich z. B. um einen Stift handeln, der zwischen den Verdickungen angeordnet ist und diese auseinanderhält, so dass sich diese nicht vom Halteelement lösen können. Die Festlegung des Implantats am Halteelement der Einführhilfe über Formschluss, der über die elektrolytische Ablösbarkeit eines Abschnitts des Halteelements kontrolliert wird, ist im Hinblick auf die sichere Steuerbarkeit der Platzierung und ggf. auch die Repositionierung oder das Zurückziehen des Implantats von Vorteil.

Eine weitere Möglichkeit besteht darin, als korrodierbar ausgebildeten Abschnitt des Halteelements eine Scheibe mit einer Öffnung zu verwenden, wobei sich die Verdickungen der Verbindungselemente durch die Öffnung erstrecken und wobei der Durchmesser der Öffnung so auf die Verdickungen abgestimmt ist, dass ein Durchtreten der Verdickungen durch die Öffnung bei intakter Scheibe ausgeschlossen ist. Erst wenn sich die Scheibe durch Anlegen einer Spannung zumindest teilweise auflöst, können die Verdickungen des Implantats aus dem Halteelement austreten.

Eine weitere Möglichkeit besteht darin, Ablösestellen als thermische Ablösestellen auszubilden. Bei einer thermischen Ablösestelle kann die Verbindung zwischen in Längsrichtung aneinandergrenzenden Abschnitten des Implantats dadurch aufgehoben werden, dass eine Erwärmung der Ablösestelle erfolgt, woraufhin diese so weich wird oder schmilzt, dass eine Abtrennung erfolgt.

Sinnvollerweise werden die Ablösemechanismen für die 1. und 2. Ablösestelle unterschiedlich gewählt. Auf diese Weise wird sichergestellt, dass, je nach Bedarf, durch unterschiedliche Maßnahmen jeweils gezielt die gewünschte Ablösestelle angesteuert wird. Möglich sind z. B. folgende Kombinationen:

| **1. Ablösestelle** | **2. Ablösestelle** |
|---|---|
| mechanisch | elektrolytisch |
| elektrolytisch | mechanisch |
| mechanisch | thermisch |
| thermisch | mechanisch |
| elektrolytisch | thermisch |
| thermisch | elektrolytisch |

Bevorzugt ist eine Kombination, bei der die 1. Ablösestelle mechanisch, die 2. Ablösestelle elektrolytisch ablösbar ausgebildet ist.

Vorzugsweise läuft das Implantat in Längsrichtung betrachtet vom Fixierabschnitt ausgehend im Übergangsabschnitt eng zusammen und erweitert sich wiederum im distalen Abschnitt. Der Querschnitt des Implantats in Längsrichtung ist somit im Übergangsabschnitt erheblich kleiner als im distalen Abschnitt oder im Fixierabschnitt. Im Übergangsabschnitt liegen lediglich ein oder mehrere Stege oder Drähte eng nebeneinander, was die Flexibilität des Implantats erhöht. Eine hohe Flexibilität ist wünschenswert, weil viele Aneurysmen keine regelmäßige Form aufweisen, sondern beispielsweise zu einer Seite abkippen. Ein flexibler Übergangsabschnitt kann dazu beitragen, dass sich der distale Abschnitt optimal an die Aneurysmaform anpasst.

Die Stege/Drähte im Übergangsabschnitt können zumindest bereichsweise durch eine Hülse verlaufen, die die Stege/Drähte im Übergangsabschnitt zusammenhält. Unter "bereichsweise" wird in diesem Zusammenhang verstanden, dass die Stege/Drähte im Übergangsabschnitt nicht unbedingt über ihre gesamte Länge durch die Hülse verlaufen müssen; es ist ausreichend, wenn ein Teil der Länge der Stege/Drähte im Übergangsabschnitt von der Hülse umgeben ist. Mit anderen Worten kann die Hülse kürzer sein als der Übergangsabschnitt und die diesen ausbildenden Stege/Drähte.

Dadurch, dass die Stege/Drähte im Übergangsabschnitt durch eine Hülse zusammengehalten werden, bleiben sie in gewissem Rahmen relativ zueinander beweglich, können jedoch gleichwohl nicht über das vorgesehene Maß hinaus radial expandieren. Es wird dadurch auch vermieden, dass sich eines oder mehrere Stege/Drähte im Übergangsabschnitt radial abspreizen. Gleichwohl ist das Implantat um den Übergangsabschnitt sehr flexibel und kann sich daher gut anpassen an die Gestalt der Blutgefäße und des Aneurysmas. Besondere Bedeutung hat dies für stark asymmetrische Aneurysmen und Aneurysmen, die zu einer Seite abkippen.

Durch eine dünne Ausgestaltung des Übergangsabschnitts wird auch gewährleistet, dass der Blutfluss zu abzweigenden Gefäßen nicht oder kaum beeinträchtigt ist. Mit anderen Worten sorgt der Fixierabschnitt bei Bedarf für eine Fixierung des Implantats im Trägergefäß, der Übergangsabschnitt für eine ausreichende Flexibilität sowie den ungehinderten Blutstrom von und zu abzweigenden Gefäßen und der distale Abschnitt für den Verschluss des Aneurysmas, wobei der distale Abschnitt entweder unmittelbar den Blutfluss in das Aneurysma minimieren kann oder dafür sorgt, dass in das Aneurysma eingeführte Okklusionsmittel im Aneurysma verbleiben.

Der distale Abschnitt kann, je nach Form des Aneurysmas und Gestalt des Implantats im Aneurysma selbst platziert werden oder auch vor dem Aneurysma, d.h. vor dem Aneurysmahals auf der Seite des Trägergefäßes. Als Platzierung im Aneurysma wird in diesem Zusammenhang auch die Platzierung im Aneurysmahals angesehen, der zum jeweiligen Aneurysma gehört. In der Regel wird der distale Abschnitt im Eintrittsbereich des Aneurysmas positioniert.

Der Begriff "Hülse" ist weit zu verstehen, d.h. es kann sich um ein kurzes Rohr, aber auch um sonstige Gestaltungen handeln, vorausgesetzt, die Hülse weist einen inneren Hohlraum auf, durch den die Stege/Drähte verlaufen können. Die Hülse kann beispielsweise die Form einer Manschette haben. Insbesondere kann es sich bei der Hülse auch um eine Drahtwendel handeln, die einen inneren Hohlraum aufweist.

Wichtig ist, dass die Hülse bei kontrahiertem Implantat nicht verrutscht und nicht in den Bereich des Fixierabschnitts oder des distalen Abschnitts gelangt. In diesem Fall könnte es nämlich dazu kommen, dass die Hülse die Expansion des Implantats nach Freisetzung behindert. Sofern ein entsprechendes Verrutschen der Hülse nicht durch die Dimensionen der einzelnen Abschnitte des Implantats ohnehin ausgeschlossen ist, kann es daher sinnvoll sein, proximal und/oder distal der Hülse Stopper vorzusehen, die ein Verschieben oder Verrutschen der Hülse über die Stopper hinaus verhindern. Bei den Stoppern kann es sich beispielsweise um radiale Erweiterungen im Übergangsabschnitt handeln.

Eine weitere Möglichkeit, ein Verrutschen der Hülse zu verhindern, besteht darin, ein oder mehrere Stege/Drähte im Übergangsabschnitt mit einer Öse zu versehen, wobei die Hülse jedenfalls abschnittsweise durch die Öse verläuft, sodass die Hülse in ihrer Beweglichkeit in Längsrichtung des Implantats eingeschränkt wird. Die Öse kann z. B. dadurch geschaffen werden, dass ein oder mehrere Stege/Drähte im Übergangsabschnitt einen größeren Querschnitt aufweisen und im Bereich des größeren Querschnitts eine Öffnung im Steg/Draht vorhanden ist. Die Öffnung ist typischerweise länglich, sodass eine gewisse Längsverschiebbarkeit der Hülse zwecks Gewährleistung einer ausreichenden Flexibilität gegeben ist, die Längsverschiebbarkeit jedoch durch das distale und das proximale Ende der Öse begrenzt wird. Die Öffnung verläuft normalerweise im Wesentlichen orthogonal zur Längsachse des Implantats. In der Regel weist der betreffende Steg bzw. Draht die Öse auf der Außenseite auf, sodass der Steg/Draht im Übrigen von der Hülse umgeben wird, wobei die Hülse durch die Öse verläuft. Denkbar ist jedoch auch der umgekehrte Fall, bei dem die Öse vom betreffenden Steg/Draht aus in Richtung Zentrum des Übergangsabschnitts weist; auch in diesem Fall verläuft die Hülse durch die Öse, der übrige Bereich des Stegs/Drahts liegt jedoch außerhalb der Hülse.

Besonders vorteilhaft ist, wenn die Hülse zumindest teilweise aus einem röntgensichtbaren Material gefertigt ist. Dies erlaubt, typischerweise zusammen mit weiteren röntgensichtbaren Markierungen am Implantat, eine Visualisierung und damit Kontrolle des Implantationsvorgangs. Bevorzugt ist in diesem Zusammenhang eine Fertigung der Hülse aus Platin, Platin-Legierungen wie Platin-Iridium oder Gold. Möglich ist eine Fertigung der Hülse, beispielsweise der Drahtwendel aus dem entsprechenden Material, denkbar ist jedoch auch ein Überzug wie z. B. in Form einer Goldbeschichtung.

Um eine gewisse Beweglichkeit der Stege/Drähte zueinander zu erhalten, ist es bevorzugt, wenn die Stege/Drähte im Übergangsabschnitt parallel zueinander verlaufen. Denkbar ist auch eine leichte Verdrillung der Stege/Drähte, diese sollte aber nicht zu steif ausfallen, um die Anpassungsfähigkeit des Implantats nicht zu gefährden.

Die Begriffe "proximal" und "distal" sind so zu verstehen, dass sie beim Einsetzen des Implantats zum behandelnden Arzt weisende Teile des Implantats bezeichnen (proximal) bzw. vom behandelnden Arzt weg weisende Teile (distal). Das Implantat wird somit typischerweise durch einen Katheter in distaler Richtung vorgeschoben. Der Begriff "axial" bezieht sich auf die von proximal nach distal verlaufende Längsachse des Implantats, der Begriff "radial" auf hierzu senkrechte Ebenen.

Am proximalen Ende des Fixierabschnitts ist dieser über die 1. Ablösestelle mit der Einführhilfe verbunden. Typischerweise laufen die Stege/Drähte des Fixierabschnitts in Richtung der 1. Ablösestelle zusammen. Die 1. Ablösestelle befindet sich vorzugsweise an der Peripherie, d. h. exzentrisch auf dem Umfang des Fixierabschnitts in seiner expandierten Form und kommt im implantierten Zustand, wenn das Implantat in seiner expandierten Form vorliegt, an der Gefäßwandung zu liegen. Möglich ist auch, den Fixierabschnitt nach proximal in mehreren, bevorzugt 2 oder 3 Kupplungselementen zusammenlaufen zu lassen, wobei diese Kupplungselemente wiederum bevorzugt im radialen Randbereich, exzentrisch auf dem Umfang des Fixierabschnitts liegen. Die Kupplungselemente wiederum sind über die 1. Ablösestelle mit der Einführhilfe verbunden. Das Vorsehen von mehreren Kupplungselementen kann die Rückziehbarkeit des Implantats verbessern. Dies gilt besonders dann, wenn der Fixierabschnitt relativ kurz ist.

Bei der Einführhilfe handelt es sich insbesondere um einen Führungsdraht. Die exzentrische Anordnung der 1. Ablösestelle am proximalen Ende des Fixierabschnitts erleichtert bei Fehlplatzierungen das Rückziehen des Implantats in den Platzierungskatheter. Darüber hinaus wird der Blutfluss durch eine exzentrisch angeordnete Ablösestelle weniger behindert, insbesondere, wenn der Fixierabschnitt dauerhaft im Blutgefäß verbleibt. Die Einführhilfe ist bevorzugt aus Edelstahl, Nitinol oder einer Cobalt-Chrom-Legierung gefertigt.

Mit dem Fixierabschnitt stützt sich das Implantat an der Gefäßwand des Blutgefäßes ab, in dem das Implantat implantiert wird, und wird dadurch fixiert. In diesem Bereich ist das Gefäß nicht geschädigt und geeignet, den einer Stentwandung ähnelnden Fixierabschnitt zu tragen. Bei selbstexpandierenden Implantaten aus einem Formgedächtnis-Material, insbesondere einem Formgedächtnis-Metall, bevorzugt einer Nickel-Titan-Legierung, legt sich der Fixierabschnitt nach der Freisetzung aus dem Katheter selbständig an die Gefäßwandung an, bei ballonplatzierbaren Implantaten wird das Implantat in diesem Bereich durch einen Platzierungsballon aufgeweitet und gegen die Gefäßwand gepresst. Bevorzugt sind selbstexpandierende Implantate. Wenn sich jedoch eine Fixierung durch den Fixierabschnitt als nicht notwendig herausstellt, kann auch eine Ablösung an der 2. Ablösestelle erfolgen und der Fixierabschnitt wird samt Einführhilfe nach Platzierung des distalen Abschnitts entfernt.

Der distale Abschnitt ist gegenüber dem Fixierabschnitt und zumeist noch stärker gegenüber dem Übergangsabschnitt radial nach außen erweitert. Er dient der Platzierung im Aneurysma selbst oder im Bereich des Zugangs zum Aneurysma, d. h. am Aneurysmahals und verschließt diesen bzw. hindert in das Aneurysma eingebrachte Okklusionsmittel am Austritt. Entscheidend ist, dass letztlich im Aneurysma eine Blutgerinnung einsetzt. Auf der einen Seite muss die Oberflächenabdeckung hinreichend groß sein, um in das Aneurysma eingebrachte Okklusionsmittel daran zu hindern, aus dem Aneurysma zu entweichen, oder durch ausreichendes Material eine dichte Oberfläche zu schaffen, auf der anderen Seite muss eine hinreichend große Flexibilität des Implantats erhalten bleiben, um es im Bereich des Bifurkationsaneurysmas einbringen zu können.

Der distale Abschnitt kann im expandierten Zustand nach radial außen weisende Streben, Schlaufen oder Bögen aufweisen. Diese dienen der Verankerung des Implantats im oder vor dem Aneurysma. Häufig zeigt daher das Implantat von distal betrachtet im distalen Abschnitt eine Blütenform. Der im expandierten Zustand radial erweiterte distale Abschnitt kann beispielsweise einen radialen Durchmesser von 2 bis 20 mm, bevorzugt 5 bis 15 mm haben. In der Regel sind wenigstens zwei Streben/Schlaufen/Bögen vorgesehen, insbesondere drei Streben/Schlaufen/Bögen oder mehr. Typischerweise beträgt die Zahl der Streben/Schlaufen/Bögen 1 bis 24, bevorzugt sind 2 bis 8. Bei den Streben, Schlaufen oder Bögen kann es sich um entsprechend geformte Drahtelemente handeln, sie können aber auch, soweit das Implantat aus einem Rohr geschnitten ist, entsprechend durch Laserschneiden des gleichen Rohrs, in der Regel gefolgt von einer Wärmebehandlung, erzeugt sein. Ein Anfügen der Streben, Schlaufen oder Bögen ist beispielsweise mittels Laserschweißen möglich. Im Falle von Schlaufen oder Bögen handelt es sich vorzugsweise um vom Übergangsabschnitt ausgehende, eine Biegung ausbildende und zurück verlaufende Drahtelemente, wobei grundsätzlich beliebig komplexe Formen für die Schlaufen/Bögen denkbar sind. Insbesondere kann es sich auch um dreidimensionale Objekte handeln, je nachdem, wie die Schlaufen oder Bögen verlaufen. Die Schlaufen bzw. Bögen sollten weitgehend atraumatisch sein und die empfindliche Gefäßwand des Aneurysmas nicht verletzen. Möglich ist aber auch der Einsatz sonstiger Filamente oder Streben, durch die eine radiale Erweiterung des distalen Abschnitts gegenüber dem Fixierabschnitt und dem Übergangsabschnitt herbeigeführt wird. Die Erweiterung kann beispielsweise trompetenförmig, korbförmig, blütenförmig oder in Form eines Geflechts vorliegen. Nach außen abstehende Streben sind vorzugsweise nach radial innen konzentrisch ausgerichtet. Gleichzeitig können die Streben in Richtung distal ragen. Beispielsweise können jeweils zwei oder mehr Streben von einem gemeinsamen Verbindungspunkt ausgehen.

Der Winkel, den die Streben/Schlaufen/Bögen zur Längsachse des Implantats im implantierten Zustand ausbilden, liegt zwischen -45° und +175°, wobei ein positiver Winkel für nach radial außen und ein negativer Winkel für nach radial innen weisende Streben/Schlaufen/Bögen steht. Im Falle von verhältnismäßig regelmäßigen Bifurkationsaneurysmen liegt der Winkel bevorzugt bei +45° bis +90°, teilweise treten jedoch auch Aneurysmen auf, die eine unregelmäßige Form aufweisen, insbesondere stark asymmetrisch sind. In solchen Fällen kann es sinnvoll sein, stark abweichende Winkel der Streben/Schlaufen/Bögen zum Einsatz zu bringen. Beispielsweise kann es sinnvoll sein, den Winkel sehr groß zu wählen, wenn die Wandung in einem Bereich des Aneurysmas stark in Richtung des zuführenden Gefäßes ausgewölbt ist. In solchen Fällen sind Winkel > 90° denkbar. In anderen Fällen kann es zweckmäßig sein, einen Teil der Streben/Schlaufen/Bögen nach innen weisen zu lassen, d. h. negative Winkel zu wählen, um sich an die Wandung des Aneurysmas anzupassen. Bei Implantaten mit sehr schmalem Übergangsabschnitt bewirkt allerdings allein die Flexibilität des Übergangsabschnitts bereits, dass sich die Streben/Schlaufen/Bögen auch ohne besonders große oder kleine voreingestellte Winkel gut an die Form des Aneurysmas anpassen können. Die Winkel können variieren, beispielsweise kann es bei einem asymmetrischen Aneurysma sinnvoll sein, einige Schlaufen mit Winkeln > 90° vorzusehen, während andere Schlaufen herkömmliche Winkel im Bereich zwischen 45° und 90° aufweisen. Wichtig ist, dass die genannten Winkel nach Implantierung ausgebildet werden, sich also auf den expandierten Zustand beziehen; auch ein Implantat, bei dem im Zustand vor der Implantierung, etwa durch äußere Zwänge, sich die angegebenen Winkel noch nicht ausgebildet haben, ist erfindungsgemäß geeignet.

Winkel, die die Streben/Schlaufen/Bögen zur Längsachse des Implantats ausbilden, können z. B. zwischen 45° und 90°, -45° und 0°, 90° und 135° oder 135° und 175° liegen.

Die Streben/Schlaufen/Bögen im distalen Abschnitt können Fortsetzungen der das übrige Implantat ausbildenden Stege/Drähte sein, es kann sich aber auch um gesonderte Filamente handeln, die im distalen Bereich des übrigen Implantatkörpers, d. h. am distalen Ende des Übergangsabschnitts festgelegt sind, beispielsweise durch Laserverschweißen. Dabei kann jede Strebe/jede Schlaufe oder jeder Bogen des distalen Abschnitts an einem oder mehreren Verbindungspunkten mit dem weiteren Implantatkörper verbunden sein, insbesondere können auch nur ein oder zwei Verbindungspunkte pro Schlaufe/Strebe/Bogen vorgesehen sein.

Alternativ zur Ausbildung des distalen Abschnitts aus Schlaufen oder Bögen kann der distale Abschnitt im expandierten Zustand auch kugelförmig, pilzförmig, ankerförmig oder ellipsoidförmig erweitert sein. Bei den genannten Formen handelt es sich um Alternativen, durch die ebenfalls ein radial erweiterter distaler Abschnitt ausgebildet werden kann. Ein kugelförmiger Abschnitt kann sich beispielsweise gut an die Innenwandungen des Aneurysmas anschmiegen, da ein regelmäßiges Bifurkationsaneurysma häufig im Wesentlichen die Form einer Kugel aufweist. Dabei sei erwähnt, dass als Kugelform im Sinne der Erfindung nicht nur exakte Kugeln entsprechend der geometrischen Definition verstanden werden, vielmehr werden auch hiervon abweichende, runde, dreidimensionale Formen erfindungsgemäß als Kugeln angesehen. In einigen Fällen ähnelt die Form des Abschnitts auch einem Ellipsoid, wobei auch hier gilt, dass das Vorliegen eines exakten Rotationsellipsoids nicht erforderlich ist, um als ellipsoidförmig im Sinne der Erfindung zu gelten. Weitere Möglichkeiten sind pilz- oder ankerförmige Abschnitte, die sich insbesondere bei unregelmäßigen Aneurysmen eignen, beispielsweise wenn die Wandung in einem Bereich des Aneurysmas stark in Richtung des zuführenden Gefäßes ausgewölbt ist. Dies wird dadurch gewährleistet, dass bei einer Pilz- oder Ankerform einige Bereiche des Abschnitts in proximaler Richtung verlaufen. Dabei sei klargestellt, dass ein pilz-oder ankerförmiger Abschnitt selbst ebenfalls asymmetrisch sein, beispielsweise nur auf einer Seite in proximaler Richtung verlaufende Bereiche aufweisen kann. Der distale Abschnitt kann lasergeschnitten oder auch geflochten sein, wobei bevorzugt 8 bis 128 Stege oder Drähte zum Einsatz kommen.

Im distalen Abschnitt kann zentral ein Bereich vorgesehen sein, der der Blockade des Aneurysmas dient, nämlich der Verhinderung des Austritts von Okklusionsmitteln und/oder der Abkopplung des distalen Abschnitts vom Blutfluss. Die hierfür vorgesehenen Elemente werden als Trennelemente bezeichnet. Der Bereich kann einerseits durch das Einziehen von Fasern, Fäden, dünnen Drähten, einer Membran oder dergleichen Trennelemente ausgebildet werden, kann aber auch integraler Teil des Implantats in dem Sinne sein, dass es sich um aus dem Ausgangsrohr herausgeschnittene und entsprechend umgeformte Trennelemente oder um aus einem Drahtgeflecht gebildete Trennelemente, etwa Schlaufen oder Stege, handelt. Im Falle von Schlaufen oder Stegen weisen diese radial nach innen in das Lumen des Implantats, im Gegensatz zu den zuvor beschriebenen Schlaufen des distalen Abschnitts, die zumindest größtenteils nach außen weisen. Damit die nach innen weisenden Schlaufen/Stege sich nicht gegenseitig behindern, kann es sinnvoll sein, diese asymmetrisch auszugestalten. Die Zahl kann variieren, abhängig von der Struktur des Implantats.

Als Trennelemente ausgebildete Fäden können aus einem Polymermaterial hergestellt sein, beispielsweise einem Polyamid wie Nylon (Polyhexamethylenadipinsäureamid). Ebenso möglich ist die Herstellung aus Metall, wobei Formgedächtnislegierungen bevorzugt sind, insbesondere Nickel-Titan-Legierungen wie Nitinol.

Eine weitere Möglichkeit besteht darin, als Trennelement eine Membran vorzusehen, die weitgehend oder vollständig undurchlässig für Blut ist und das Aneurysma auf diese Weise vom Blutstrom abkoppelt. Sofern eine weitgehend vollständige Abkopplung vom Blutstrom erreicht wird, ist eine Einbringung von Okklusionsmitteln in das Aneurysma u. U. entbehrlich, d. h. das Trennelement dient in diesem Fall nicht dem Zurückhalten von Okklusionsmitteln. Die Membran kann an den Streben/Schlaufen/Bögen fixiert und/oder auf ein Geflecht aus Fäden oder Drähten aufgespannt sein, bspw. können Fäden oder Drähte eine Struktur bilden, über oder auf die die Membran aufgespannt ist. Zusätzlich sind weitere, z. B. kreuzförmig verlaufende Fäden/Drähte denkbar, die ein Fadenkreuz ausbilden. Fäden oder Drähte sind jedoch nicht unbedingt erforderlich, auch ein Überspannen des zentralen Bereichs des distalen Abschnitts ohne zusätzliche Fäden/Drähte ist möglich.

Ein Vorteil der Verwendung einer Membran als Trennelement ist darin zu sehen, dass diese sich bei der Platzierung des Implantats im Katheter eng nach distal oder proximal zusammenlegt, so dass ein Implantat mit im expandierten Zustand weitgehend dichtem Trennelement zur Verfügung gestellt wird, das sich im kontrahierten Zustand auch durch enge Blutgefäße gut hindurchführen lässt. Im Übrigen bleibt der Aufbau des Implantats im Vergleich zu einem Implantat ohne Trennelement weitgehend unverändert.

Auch in den Fällen, in denen als Trennelement eine Membran vorgesehen ist, kann es jedoch von Vorteil sein, zusätzlich Okklusionsmittel in das Aneurysma einzubringen. Aus diesem Grund kann es sinnvoll sein, eine Membran mit einer oder mehreren Ausnehmungen zu verwenden, so dass durch die Ausnehmung ein Einbringen von Okklusionsmitteln, insbesondere Coils möglich ist. Die Ausnehmung sollte eine Größe aufweisen, dass ein Katheter durch die Ausnehmung in den Bereich des Aneurysmas vorgeschoben werden kann, wobei durch den Katheter die Okklusionsmittel eingebracht werden. Andererseits sollte der Aneurysmahals so weit abgedeckt sein, dass die Okklusionsmittel das Aneurysma nicht unkontrolliert verlassen können, wobei ggf. den Bereich der Membran überspannende Fäden/Drähte eine zusätzliche Rückhaltefunktion erfüllen. Selbstverständlich dürfen in diesem Fall die Fäden bzw. Drähte nur so dicht verlaufen, dass ein Hindurchführen eines Katheters und ein Einbringen von Okklusionsmitteln weiterhin möglich ist.

Um Okklusionsmittel in das Aneurysma einzubringen, kann die Membran auch partiell durchstoßbar ausgebildet werden, wobei zum Durchstoßen typischerweise ein Mikrokatheter oder ein Führungsdraht verwendet wird. Durch die so geschaffene Öffnung wird sodann ein Mikrokatheter geführt, durch den die Okklusionsmittel eingeschoben werden. Die Membran sollte so beschaffen sein, dass sie auch nach Durchstoßen partiell erhalten bleibt, so dass die Okklusionsmittel weiterhin durch die Membran am Wiederaustritt gehindert werden. Beispielsweise können als zusätzliche Trennelemente vorgesehene Fäden oder Drähte, die in der Form eines Fadenkreuzes aufgespannt sein können, dafür sorgen, dass lediglich ein Segment der Membran beim Durchstoßen eine Öffnung ausbildet, während die übrigen Segmente von der Membran abgedeckt bleiben, da die Randbereiche der Membran von den Fäden/Drähten stabilisiert und vor einem Einreißen geschützt werden. Bei der als Trennelement vorgesehenen Membran kann es sich um eine einzelne Membran handeln, die nur partiell durchstoßen wird, oder auch um mehrere kleine Membranen.

Statt oder zusätzlich zum Vorsehen einer Membran als Trennelement kann es sinnvoll sein, Membranen im Inneren der (Draht)schlaufen oder Bögen des distalen Abschnitts vorzusehen. Membranen können auch zwischen Streben des distalen Abschnitts vorgesehen sein. Auch kugel-, pilz-, anker- oder ellipsoidförmig ausgebildete distale Abschnitte können mit einer Membran bespannt sein. Die Membranen können zum einen bei Platzierung vor oder im Eintrittsbereich des Aneurysmas der Umlenkung des Blutstromes in abzweigende Gefäße, zum anderen der Unterbindung des Blutstromes in das Aneurysma dienen.

Die Membran muss nicht auf das Trennelement und das Innere der Schlaufen/Bögen beschränkt sein, sondern kann insgesamt den distalen Abschnitt überspannen, wobei die Streben, Schlaufen oder Bögen der Fixierung der Membran dienen können. Beispielsweise können Membranen in den Zwischenräumen zwischen den Streben, Schlaufen oder Bögen vorgesehen sein.

Auch wenn der distale Abschnitt ganz oder teilweise von anderen Filamenten als Schlaufen gebildet wird, ist es möglich, hier Membranen vorzusehen. Beispielsweise können eine oder mehrere Membranen von radial nach außen abstehenden Streben aufgespannt werden. In diesem Fall ähnelt der Aufbau dem eines Schirms, d. h. beim Expandieren des distalen Abschnitts spannen die sich entfaltenden Streben eine durchgehende oder mehrere Membranen zwischen sich auf. Durch das Vorsehen von mehreren Streben und einer entsprechenden Zahl an Strebenenden wird erreicht, dass die von der Membran abgedeckte Fläche größer und kreisähnlicher wird und weniger große Zwischenräume aufweist.

Zur Abgrenzung und Verstärkung der Membran können auch zwischen den einzelnen Streben/Schlaufen/Bögen gespannte Fäden dienen, d. h. die Membranen werden zumindest teilweise seitlich von einem oder mehreren Fäden begrenzt, die die Streben/Schlaufen/Bögen miteinander verbinden. Dabei muss die Begrenzung der jeweiligen Membran nicht in jeder Richtung über einen Faden erfolgen, hierzu können auch teilweise die Streben/Schlaufen/Bögen selbst dienen. Beispielsweise kann der äußere Rand der Membran, der häufig auch weiter distal liegt, durch Fäden, der innere Rand durch Streben/Schlaufen/Bögen umrandet sein. Im Vergleich zu einer Membran ohne seitliche Begrenzung wird insbesondere ein zusätzlicher Schutz der Membran gegen Beschädigungen und Risse erreicht. Die Fäden werden bevorzugt aus einem Polyamid wie Nylon gefertigt.

Die Membran (unabhängig davon, ob als Trennelement verwendet oder in anderen Bereichen des distalen Abschnitts angeordnet) kann aus einem Polymermaterial wie Polytetrafluorethylen, Polyester, Polyamiden, Polyurethanen oder Polyolefinen hergestellt sein. Besonders bevorzugt sind Polycarbonaturethane. Wünschenswert ist insbesondere eine integrale Verbindung der Membran mit ggf. als weitere Trennelemente vorgesehenen Fäden oder Drähten. Diese kann durch eine Tauch- oder Sprühbeschichtung der Fäden/Drähte herbeigeführt werden.

Vorzugsweise wird die Membran durch Elektrospinnen erzeugt. Hierbei werden Fibrillen bzw. Fasern aus einer Polymerlösung mit Hilfe von elektrischem Strom auf einem Substrat abgeschieden. Bei der Abscheidung verkleben die Fibrillen zu einem Vlies. In der Regel haben die Fibrillen einen Durchmesser von 100 bis 3000 nm. Durch Elektrospinnen gewonnene Membranen sind sehr gleichmäßig ausgebildet und können eine Grundstruktur aus Fäden oder Drähten in sich einzuschließen. Die Membran ist zäh und mechanisch belastbar und kann mechanisch durchstoßen werden, ohne dass die Öffnung zum Ansatzpunkt für weitergehende Risse wird. Die Dicke der Fibrillen wie auch der Grad der Porosität kann durch Auswahl der Verfahrensparameter gesteuert werden. Im Zusammenhang mit der Schaffung der Membran und den dafür geeigneten Materialien wird insbesondere auf die WO 2008/049386 A1, die DE 28 06 030 A1 und die darin genannte Literatur hingewiesen.

Vorteilhaft ist auch ein Implantat, bei dem als Trennelement eine an der Innenseite des Implantats anliegende Membran verwendet wird, wobei diese Membran wiederum mit weiteren äußeren Membranabschnitten fest verbunden ist, die die einzelnen Schlaufen oder Bögen des distalen Abschnitts ausfüllen. Eine solche Membranstruktur lässt sich über Elektrospinnen erzeugen. Die inneren und äußeren Membranabschnitte sind in diesem Fall teilweise verbunden; dort, wo der innere Membranabschnitt keine Verbindung mit dem äußeren Membranabschnitt aufweist, zieht er sich ähnlich einem Nylonstrumpf zusammen, sodass sich eine Öffnung für die Einbringung von Okklusionsmitteln ergibt.

Statt durch Elektrospinnen kann die Membran auch über einen Tauchprozess hergestellt werden.

Eine als Trennelement dienende Membran muss nicht in jedem Fall (im expandierten Zustand) in einer Ebene orthogonal zur Längsachse des Implantats verlaufen, sondern kann auch im expandierten Zustand eine Ausrichtung in Richtung proximal aufweisen. In diesem Fall ist die Membran zwar in ihrem Randbereich im Umfang des Implantats fixiert, der mittlere Bereich der Membran erstreckt sich aber in proximaler Richtung. Es ergibt sich somit insgesamt eine Kegel- oder Pyramidenform, wobei die Basis des Kegels/der Pyramide orthogonal zur Längsachse orientiert und die Membran im Randbereich mit dem Implantat verbunden ist, während der Scheitel des Kegels/der Pyramide weiter proximal liegt. Der Blutstrom wird, wenn er auf die Membran trifft, auf diese Weise geteilt und seitwärts abgelenkt, so dass der Blutstrom in das Aneurysma weitgehend zum Erliegen kommt.

Auch im Falle einer Kegel-/Pyramidenform der als Trennelement vorgesehenen Membran kann diese eine oder mehrere Ausnehmungen aufweisen, damit weiterhin nach Platzierung des Implantats durch die Ausnehmung hindurch Okklusionsmittel in das Aneurysma eingebracht werden können.

Um die Kegel-/Pyramidenform der Membran dauerhaft zu erhalten, sollte die Membran auf einer Gerüststruktur aus Fäden oder Drähten fixiert sein, wobei es sich prinzipiell auch um Stege handeln kann, die aus der das Implantat ausbildenden Struktur z. B. mit Hilfe eines Lasers herausgeschnitten sind. Dabei sollte darauf geachtet werden, dass die Fäden/Drähte eine hinreichende Steifigkeit aufweisen, um eine Umorientierung oder Umstülpung der Membran aufgrund des Blutdrucks zu verhindern. Ggf. müssen hierfür zusätzliche Fäden oder Drähte eingebracht werden.

Eine Möglichkeit besteht darin, ein Fadenkreuz aus zwei relativ langen Einzelfäden zu erzeugen, an dem die Membran fixiert ist, wobei aufgrund der Länge der Einzelfäden die Membran zunächst nicht gespannt ist. Ein oder mehrere Fäden können darüber hinaus an einer weiter proximal liegenden Schlaufe des Implantats fixiert sein, so dass das Fadenkreuz und damit die Membran in Richtung proximal gespannt werden, sobald das Implantat gestreckt wird. Selbstverständlich muss es sich nicht um lediglich zwei Fäden handeln, die ein Fadenkreuz ausbilden, ebenso denkbar sind nahezu beliebige andere Fadengeflechte, die eine Art Gerüststruktur ausbilden, um der Membran eine Struktur aufzuprägen.

Allgemein ist für die Erfindung wesentlich, dass der distale Abschnitt, ggf. mit dem Trennelement, seine Funktion erfüllt, nämlich in das Aneurysma eingeführte Okklusionsmittel, etwa Okklusionsspiralen, zuverlässig zurückzuhalten oder den Blutstrom so abzulenken, dass weitere Okklusionsmittel unnötig sind. Das Trennelement weist bei expandiertem Implantat zumindest auch eine Komponente orthogonal zur Längsachse des Implantats auf.

Werden die Trennelemente durch das Einziehen von Fasern, Fäden oder dünnen Drähten ausgebildet, ist die Anordnung von Ösen im distalen Abschnitt zweckmäßig, in die die Fäden kreuz- oder sternförmig eingeknotet werden. Die Ösen selbst können aus einem Fasermaterial gefertigt sein. Die Fäden/Fasern bestehen z. B. aus einem geeigneten Polymer wie einem Polyamid (Nylon) oder es handelt sich um metallische Fasern.

Als Trennelemente können aber auch aus einem Rohrmaterial geschnittene Bögen oder (Draht)schlaufen genutzt werden, die in den Implantatkörper hinein umgebogen sind. Mindestens ein Bogen/eine Schlaufe ist hierfür erforderlich. Bei zwei bis vier Bögen/Schlaufen bilden diese ein stabiles Trennelement, das in ein Aneurysma eingebrachte Okklusionsmittel zuverlässig zurückhält.

Beim Kontrahieren des Implantats werden die Schlaufen typischerweise nach proximal gestreckt und legen sich an die weiteren Elemente des Implantats an, so dass sich das Implantat problemlos durch einen Katheter bewegen lässt. Zwischen den Schlaufen können schlitzförmige Öffnungen belassen werden, durch die Okklusionsmittel in das Aneurysma einführbar sind. Alternativ ist es aber auch möglich, die Schlaufen und/oder die Zwischenbereiche zwischen den Schlaufen mit einer Membran zu versehen, um ein möglichst dichtes Trennelement zu erzeugen. Auch die Verwendung von Membranen, die noch ein oder mehrere Öffnungen aufweisen, ist grundsätzlich möglich.

Zu den verschiedenen Möglichkeiten der Ausgestaltung der Trennelemente oder zum Versehen des distalen Abschnitts mit Membranen sei auch auf die WO 2014/029835 A1 verwiesen, deren Inhalt auch Gegenstand der Offenbarung der vorliegenden Erfindung sein soll.

Der distale Abschnitt des erfindungsgemäßen Implantats ist insbesondere atraumatisch, weich und elastisch ausgebildet. Die Wandungen von Aneurysmen sind empfindlich und können bei Belastung reißen, was verhindert werden muss. Entsprechend sollte insbesondere der distale Abschnitt des erfindungsgemäßen Implantats atraumatisch gestaltet sein. Dies wird beispielsweise mit der Anordnung von Schlaufen oder Bögen erreicht, die sich dort, wo sie Kontakt mit der Aneurysmawandung bekommen, sanft an diese anschmiegen. Solche Schlaufen oder Bögen können, wie andere Bereiche des Implantats auch, durch Laserschneiden aus einem Rohr generiert, mittels angefügter Drähte oder aus einem einheitlichen Drahtgeflecht erzeugt werden. Im distalen Abschnitt sollten alle Drahtenden atraumatisch ausgebildet werden, um eine Perforation der Aneurysmawand zu verhindern.

Die erfindungsgemäßen Implantate weisen in der Regel röntgendichte Markerelemente auf, die die Visualisierung und Platzierung am Implantationsort erleichtern. Beispielsweise kann es sich bei einer im Übergangsabschnitt vorgesehenen Hülse um ein solches Markerelement handeln. Darüber hinaus können Markerelemente beispielsweise im Bereich des distalen Endes des distalen Abschnitts angeordnet sein, wobei sie bei zusammengeführten Drähten die Verbindungspunkte atraumatisch umformen können. Solche Markerelemente können auch in Form von Drahtwicklungen, als Manschetten und als geschlitzte Rohrabschnitte vorliegen, die am Implantat festgelegt werden. Beispielsweise können als Markerelemente die die Schlaufen ausbildenden Filamente umgebende Markercoils vorgesehen sein, wobei in der Regel nicht die gesamten Schlaufen, sondern beispielsweise nur eine Hälfte von Markercoils umgeben ist. Für die Markerelemente kommen als Materialien insbesondere Platin und Platinlegierungen in Frage, beispielsweise eine Legierung aus Platin und Iridium, wie sie vielfach im Stand der Technik für Markerzwecke und als Material für Okklusionscoils eingesetzt wird. Andere verwendbare röntgendichte Metalle sind Tantal, Gold und Wolfram. Idealerweise ist der distale Abschnitt und sind insbesondere die Schlaufen/Streben/Bögen im distalen Abschnitt vollständig oder teilweise röntgendicht, d. h. röntgensichtbar ausgebildet. Eine weitere Möglichkeit besteht in der Beschichtung oder Füllung der Stege/Drähte mit einem röntgendichten Material.

Möglich ist es auch, röntgensichtbare Substanzen in die Membranen einzubringen. Hierbei kann es sich um röntgensichtbare Partikel handeln, wie sie üblicherweise in der Röntgentechnik als Kontrastmittel eingesetzt werden. Solche röntgendichten Substanzen sind beispielsweise Schwermetallsalze wie Bariumsulfat oder lodverbindungen. Die Röntgensichtbarkeit der Membran ist bei der Einbringung und Lokalisierung des Implantats hilfreich und kann zusätzlich oder anstelle von Markerelementen Verwendung finden. Eine weitere Alternative besteht in einer teilweisen Goldbeschichtung von Bereichen des Implantats, beispielsweise der Schlaufen oder bestimmter Bereiche der Schlaufen.

Ggf. kann ein Teil des Implantats von Stegen oder Drähten mit dünnerem Querschnitt gebildet werden, um die Flexibilität des Implantats zu erhöhen. Der Bereich liegt vorzugsweise im Fixierabschnitt und soll einem nicht regelmäßigen Verlauf des Blutgefäßes in der Fixierzone Rechnung tragen.

Das Implantat kann auf an und für sich bekannte Weise beschichtet sein. Als Beschichtungsmaterialien kommen insbesondere solche in Frage, wie sie für Stents beschrieben sind, etwa mit antiproliferativen, entzündungshemmenden, antithrombogenen, das Einwachsen fördernden und/oder die Anlagerung hindernden, hämokompatiblen Eigenschaften.

Die erfindungsgemäße Vorrichtung kann insbesondere im neurovaskulären Bereich eingesetzt werden, möglich ist jedoch auch der Einsatz im kardiovaskulären oder peripheren Bereich.

Beschrieben wird auch ein Verfahren zur Einbringung des erfindungsgemäßen Implantats in das Blutgefäßsystem. Dies kann mit Hilfe eines üblichen Mikrokatheters erfolgen; diese Technik ist allgemein erprobt und wird vielfach angewandt. Sofern das Implantat nicht bereits selbst für eine ausreichende Abdichtung des Aneurysmahalses sorgt, werden nach Platzierung des Implantats Okklusionsmittel in das Aneurysma eingebracht. Hierzu wird das distale Ende eines Katheters in das Aneurysma geführt und die Okklusionsmittel, insbesondere Coils werden eingebracht. Zum Einbringen der Okklusionsmittel kann der Katheter durch das Implantat, insbesondere durch den Innenraum des Fixierabschnitts in das Aneurysma vorgeschoben werden, wobei der Fixierabschnitt für eine gewisse Führung des Katheters sorgt. Anschließend wird der Katheter zurückgezogen; die Okklusionsmittel werden durch das Implantat daran gehindert, aus dem Aneurysma zu entweichen. Neben herkömmlichen Okklusionsmitteln wie Coils können auch in anderer Weise geformte Körper zum Verschließen von Aneurysmen eingebracht werden, beispielsweise geflochtene oder auf andere Weise geformte Kugelkörper. Unabhängig davon, ob zusätzliche Okklusionsmittel in das Aneurysma eingebracht werden, erfolgt schließlich eine Ablösung an der 1. oder 2. Ablösestelle und die Einführhilfe wird mit oder ohne den Fixierabschnitt aus dem Blutgefäßsystem zurückgezogen, während der distale Abschnitt sowie ggf. der Fixierabschnitt im Blutgefäß verbleibt.

Die Erfindung wird durch die beiliegenden Abbildungen beispielhaft näher erläutert. Es zeigen:
- Figur 1: Ein Bifurkationsaneurysma in einer Prinzipskizze;
- Figur 2a: ein erfindungsgemäßes Implantat in der Seitenansicht mit kurzem Fixierabschnitt;
- Figur 2b: ein erfindungsgemäßes Implantat in der Seitenansicht mit langem Fixierabschnitt;
- Figur 3a: ein eingesetztes Implantat vor Ablösung an der 1. Ablösestelle;
- Figur 3b: das Implantat aus Figur 3a nach Ablösung an der 1. Ablösestelle;
- Figur 4a: ein eingesetztes Implantat vor Ablösung an der 2. Ablösestelle;
- Figur 4b: das Implantat aus Figur 4a nach Ablösung an der 2. Ablösestelle;
- Figur 5a: ein eingesetztes Implantat mit Membran vor Ablösung an der 2. Ablösestelle;
- Figur 5b: das Implantat aus Figur 5a nach Ablösung an der 2. Ablösestelle.

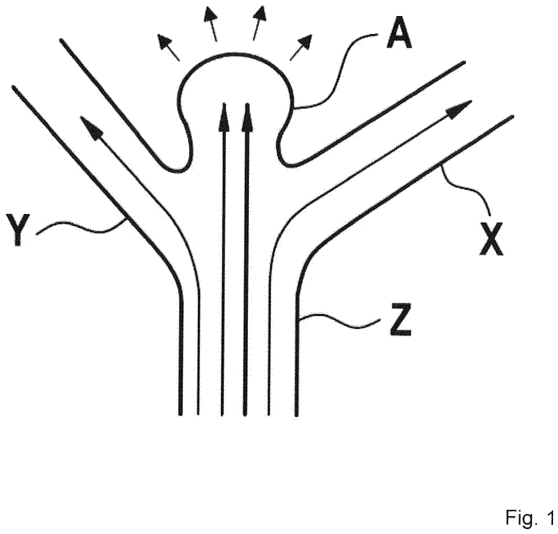

Figur 1 zeigt ein Bifurkationsaneurysma mit einem zuführenden Gefäß Z, zwei abführenden Gefäßen X und Y sowie dem in der Gabelung angeordneten Aneurysma A. Die langen Pfeile stellen den Blutstrom dar, der auf der Prallseite in das Aneurysma A einfließt und dort einen Druck nach außen ausübt, unter dem das Aneurysma sich erweitert (kleine Pfeile).

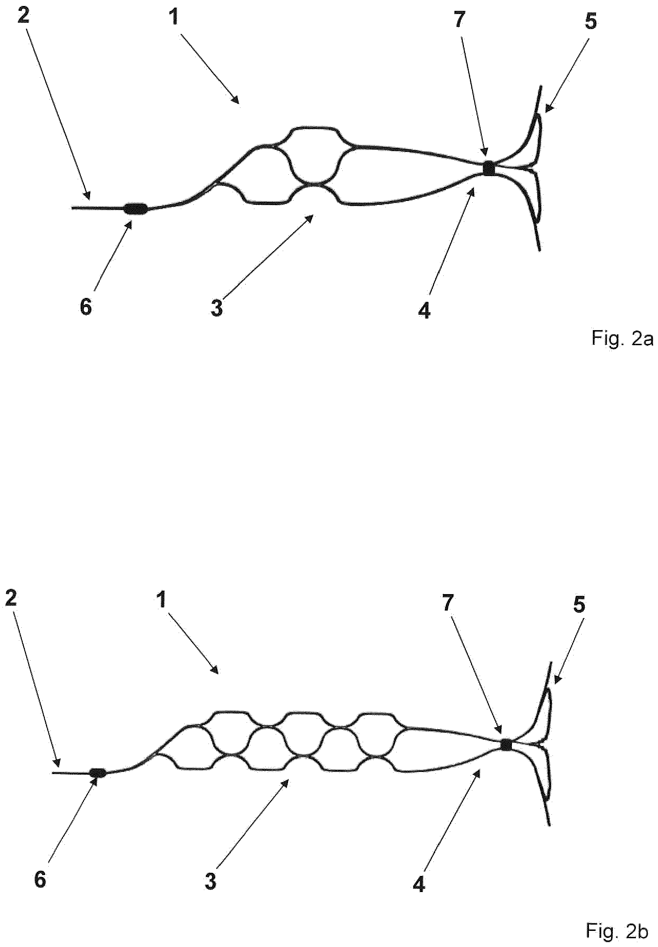

Figur 2a zeigt ein erfindungsgemäßes Implantat 1 im expandierten Zustand in der Seitenansicht. Das Implantat 1 weist einen Fixierabschnitt 3 und einen distalen Abschnitt 5 auf, wobei der distale Abschnitt 5 radial gegenüber dem Fixierabschnitt 3 erweitert ist. Dabei bildet er mehrere Schlaufen aus, die sich im Inneren des Aneurysmas an dessen Wandung anlegen.

Zwischen dem Fixierabschnitt 3 und dem distalen Abschnitt 5 ist ein Übergangsabschnitt 4 vorgesehen, der einen kleinen Querschnitt aufweist. Von proximal (in der Zeichnung links) nach distal (in der Zeichnung rechts) gesehen werden die das Implantat 1 ausbildenden Stege vom Fixierabschnitt 3 ausgehend im Übergangsabschnitt 4 eng zusammengeführt und erweitern sich anschließend wieder zum distalen Abschnitt 5. Der Fixierabschnitt 3 ist über eine 1. Ablösestelle 6 mit einer Einführhilfe 2, in der Regel einem Führungsdraht verbunden. Der Übergangsabschnitt 4 weist eine 2. Ablösestelle 7 auf.

In Figur 2b ist ein erfindungsgemäßes Implantat 1 dargestellt, das weitgehend dem aus Figur 2a entspricht, jedoch einen längeren Fixierabschnitt 3 aufweist.

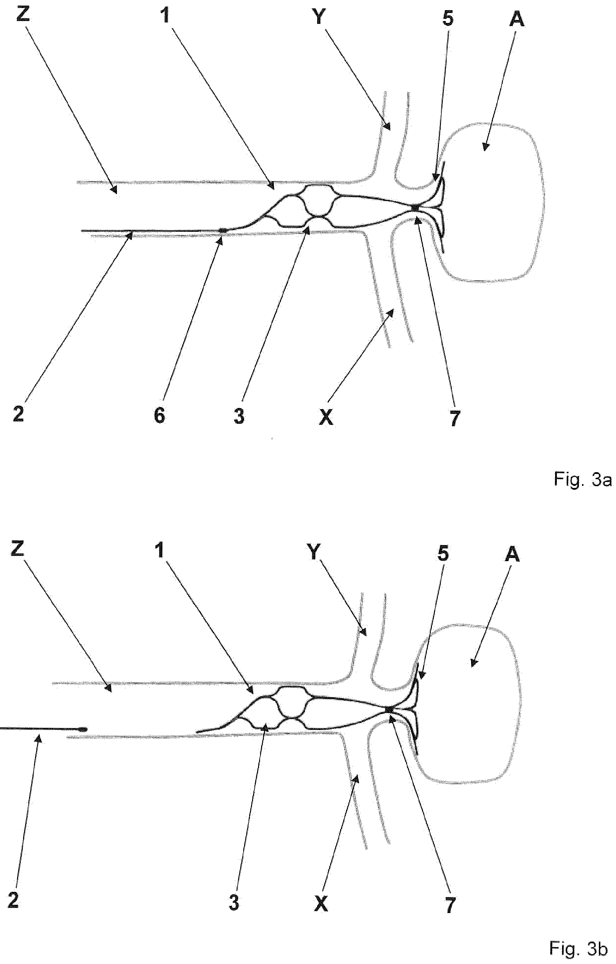

In Figur 3a ist die Platzierung des Implantats 1 aus Figur 2a im Bereich eines Bifurkationsaneurysmas A dargestellt. Das Aneurysma A liegt im Bereich der Verzweigung des Blutgefäßes Z in die Blutgefäße X und Y. Das Implantat 1 wird so platziert, dass der distale Abschnitt 5 im Eintrittsbereich des Aneurysmas A zu liegen kommt, während der Fixierabschnitt 3 für die Fixierung im Blutgefäß Z sorgt.

In Figur 3b ist die entsprechende Situation nach Ablösung des Implantats 1 an der 1. Ablösestelle 6 dargestellt, d.h. in diesem Fall verbleibt der distale Abschnitt 5 zusammen mit dem Fixierabschnitt 3 im Blutgefäßsystem. Die 2. Ablösestelle 7 bleibt unversehrt.

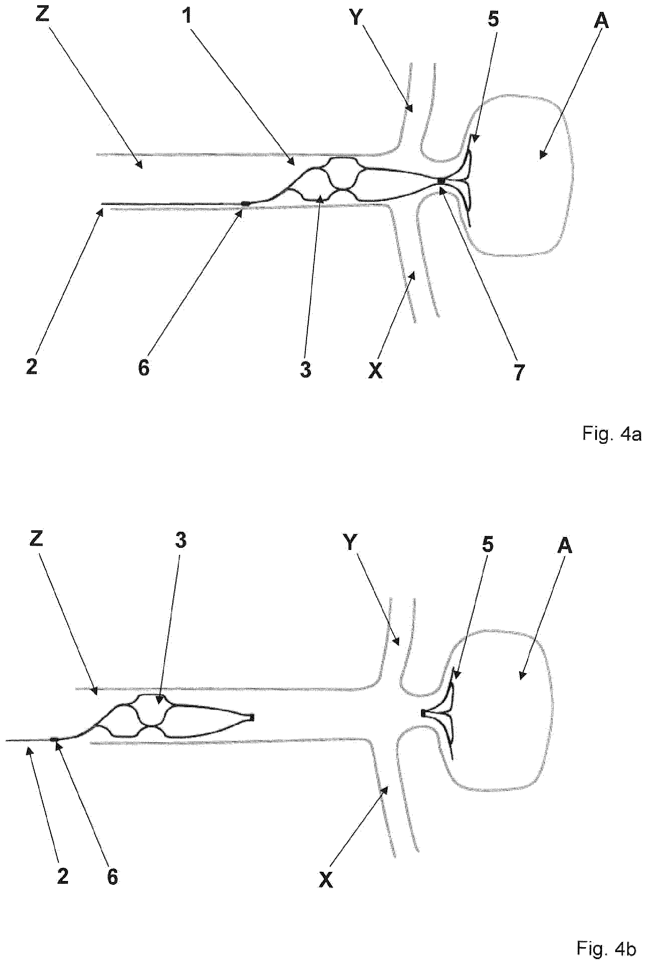

In Figur 4a ist eine der Situation aus Figur 3a entsprechende Situation dargestellt, bei der jedoch der behandelnde Arzt entscheidet, dass der distale Abschnitt 5 allein bereits ausreichend im Aneurysma A fixiert ist, d.h. eine zusätzliche Fixierung durch den Fixierabschnitt 3 nicht notwendig ist. In diesem Fall erfolgt, wie in Figur 4b dargestellt, die Abtrennung an der 2. Ablösestelle 7. Die Einführhilfe 2 wird zusammen mit dem Fixierabschnitt 3 aus dem Blutgefäßsystem entfernt; die 1. Ablösestelle 6 bleibt unversehrt.

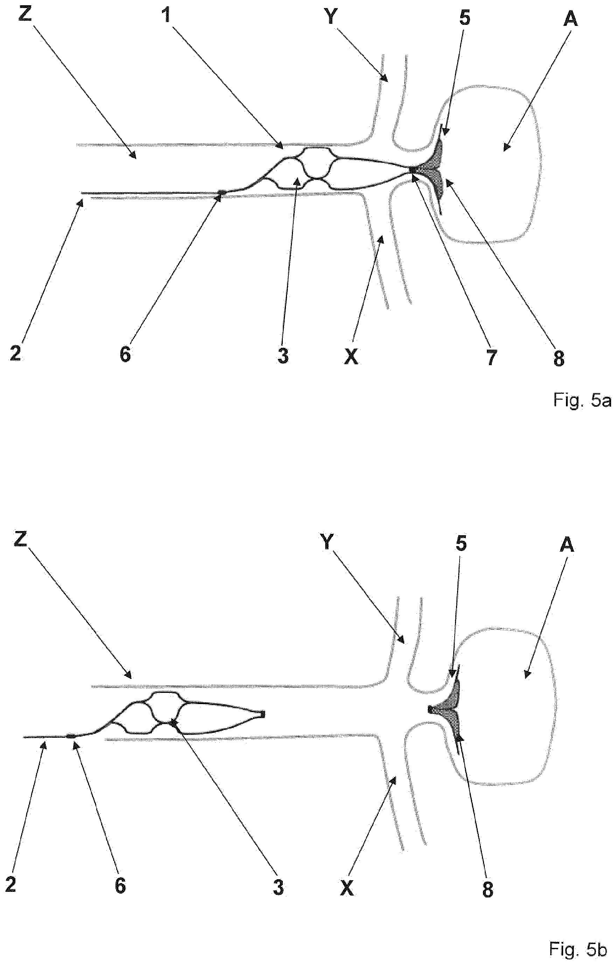

Die Figuren 5a und 5b entsprechen im vollem Umfang den Figuren 4a und 4b, jedoch weist der distale Abschnitt 5 in diesem Fall eine Bespannung mit einer Membran 8 auf. Diese sorgt für eine zusätzliche Unterbindung des Blutflusses in das Aneurysma A.

## Patentansprüche

1. Implantat zum Einsatz bei der Okkludierung von Aneurysmen (A) in Blutgefäßen im Bereich von Gefäßverzweigungen, insbesondere Bifurkationsaneurysmen (A), wobei das Implantat (1) in einem expandierten Zustand, in dem es im Blutgefäß implantiert wird, und in einem kontrahierten Zustand vorliegt, in dem es durch das Blutgefäß bewegbar ist, wobei das Implantat (1) einen proximalen Fixierabschnitt (3), mit dem das Implantat (1) an einer Gefäßwand des Blutgefäßes fixierbar ist, einen distalen Abschnitt (5), in dem das Implantat (1) im expandierten Zustand gegenüber dem Fixierabschnitt (3) radial erweitert ist und der zur Platzierung im oder vor dem Aneurysma (A) bestimmt ist, und einen Übergangsabschnitt (4) zwischen dem Fixierabschnitt (3) und dem distalen Abschnitt (5) aufweist, wobei der Fixierabschnitt (3) über eine 1. Ablösestelle (6) ablösbar mit einer Einführhilfe (2) verbunden ist, wobei der Fixierabschnitt (3) des Implantats (1) aus miteinander verbundenen oder sich kreuzenden Stegen oder Drähten aufgebaut ist,
**dadurch gekennzeichnet, dass** im Übergangsabschnitt (4) ein oder mehrere vom Fixierabschnitt (3) oder distalen Abschnitt (5) ausgehende Stege oder Drähte zentral zusammenkommen, der Übergangsabschnitt (4) eine einzelne 2. Ablösestelle (7) aufweist, die eine Abtrennung des distalen Abschnitts (5) ermöglicht und sich die Ablösemechanismen für die 1. Ablösestelle (6) und die 2. Ablösestelle (7) voneinander unterscheiden.

2. Implantat nach Anspruch 1, **dadurch gekennzeichnet, dass** die Stege oder Drähte im Übergangsabschnitt (4) zumindest bereichsweise durch eine Hülse verlaufen.

3. Implantat nach Anspruch 1 oder 2, **dadurch gekennzeichnet, dass** die 2. Ablösestelle (7) elektrolytisch ablösbar ist.

4. Implantat nach Anspruch 3, **dadurch gekennzeichnet, dass** das Implantat (1) proximal der 2. Ablösestelle (7) ganz oder teilweise mit einer elektrisch isolierenden Beschichtung versehen ist.

5. Implantat nach einem der Ansprüche 1 bis 4, **dadurch gekennzeichnet, dass** der distale Abschnitt (5) eine Mehrzahl von Streben, Schlaufen oder Bögen aufweist, die im expandierten Zustand zumindest teilweise nach radial außen weisen.

6. Implantat nach Anspruch 5, **dadurch gekennzeichnet, dass** die Streben, Schlaufen oder Bögen im expandierten Zustand zur Längsachse des Implantats (1) einen Winkel zwischen -45° und +175°, vorzugsweise zwischen +45° und +90° ausbilden, wobei ein positiver Winkel für nach radial außen und ein negativer Winkel für nach radial innen weisende Streben, Schlaufen oder Bögen steht.

7. Implantat nach Anspruch 5 oder 6, **dadurch gekennzeichnet, dass** die Schlaufen oder Bögen im Inneren mit einer Membran (8) versehen sind oder zwischen den Streben eine Membran (8) aufgespannt ist.

8. Implantat nach einem der Ansprüche 1 bis 4, **dadurch gekennzeichnet, dass** der distale Abschnitt (5) im expandierten Zustand kugelförmig, pilzförmig, ankerförmig oder ellipsoidförmig radial erweitert ist.

9. Implantat nach einem der Ansprüche 1 bis 8, **dadurch gekennzeichnet, dass** im distalen Abschnitt (5) zentral ein oder mehrere Trennelemente angeordnet sind, die im implantierten Zustand den Hals des Aneurysmas (A) zumindest teilweise verschließen.

10. Implantat nach Anspruch 9, **dadurch gekennzeichnet, dass** die Trennelemente aus Fasern, Fäden, Drähten oder Membranen (8) gebildet werden.

11. Implantat nach Anspruch 10, **dadurch gekennzeichnet, dass** sich die Membran (8) im expandierten Zustand in proximaler Richtung erstreckt und vorzugsweise eine Kegel- oder Pyramidenform ausbildet.

12. Implantat nach Anspruch 10 oder 11, **dadurch gekennzeichnet, dass** die Membran (8) eine oder mehrere Öffnungen aufweist oder in der Membran (8) eine oder mehrere Öffnungen mittels Durchstechen erzeugbar sind.

## Claims

1. Implant to be used for the occlusion of aneurysms (A) in blood vessels in the region of vascular branches, in particular bifurcation aneurysms (A), with the implant (1) being in an expanded state in which it is implanted in the blood vessel and in a contracted state in which it is movable through the blood vessel, with the implant (1) having a proximal fixing section (3) by means of which the implant (1) can be secured to the wall of a blood vessel, a distal section (5) where the implant (1) in expanded state is radially widened relative to the fixing section (3) and which is intended for placement in or in front of the aneurysm (A), and having a transition section (4) located between the fixing section (3) and the distal section (5), wherein the fixing section (3) being detachably connected to an insertion aid (2) via a 1st detachment point (6), wherein the fixing section (3) of the implant (1) is composed of interconnected or intersecting struts or wires,
**characterized in that** in the transition section (4) one or several struts or wires originating from the fixing section (3) or the distal section (5) converge centrally, the transition section (4) is provided with a single 2nd detachment point (7) that enables the distal section (5) to be separated, and the detachment mechanisms for the 1st detachment point (6) and the 2nd detachment point (7) differ from one another.

2. Implant according to claim 1, **characterized in that** the struts or wires in the transition section (4) extend at least to some extent through a sleeve.

3. Implant according to claim 1 or 2, **characterized in that** the 2nd detachment point (7) is detachable electrolytically.

4. Implant according to claim 3, **characterized in that** the implant (1) is provided in whole or in part with an electrically insulating coating proximal to the 2nd detachment point (7).

5. Implant according to any of claims 1 to 4, **characterized in that** the distal section (5) comprises a plurality of struts, loops or curves that in expanded state at least partially are facing radially outward.

6. Implant according to claim 5, **characterized in that** the struts, loops or curves in expanded state form an angle ranging between -45° and +175°, preferably between +45° and +90° in relation to the longitudinal axis of the implant (1), wherein a positive angle stands for struts, loops or curves pointing radially outwards and a negative angle for struts, loops or curves pointing radially inwards.

7. Implant according to claim 5 or 6, **characterized in that** the loops or curves are provided inside with a membrane (8) or that a membrane (8) is spanned between the struts.

8. Implant according to any of claims 1 to 4, **characterized in that** the distal section (5) is radially widened in expanded state so as to form a spherical, mushroom, anchor, or ellipsoidal shape.

9. Implant according to any of claims 1 to 8, **characterized in that** one or several separation elements are arranged centrally in the distal section (5), said separation elements at least partially occluding the neck of the aneurysm (A) in the implanted state.

10. Implant according to claim 9, **characterized in that** the separation elements are formed from fibers, threads, wires or membranes (8).

11. Implant according to claim 10, **characterized in that** the membrane (8) in expanded state extends in proximal direction and, preferably, has a conical or pyramid form.

12. Implant according to claims 10 or 11, **characterized in that** the membrane (8) has one or several openings or that in the membrane (8) one or several openings can be produced by a piercing method.

## Revendications

1. Implant destiné à être utilisé lors de l'occlusion d'anévrismes (A) dans des vaisseaux sanguins dans la zone de ramifications de vaisseaux, en particulier d'anévrismes sur une bifurcation (A), dans lequel l'implant (1) peut être déplacé dans un état expansé, dans lequel il est implanté dans le vaisseau sanguin, et un état contracté, dans lequel il peut être déplacé à travers le vaisseau sanguin, dans lequel l'implant (1) présente une section de fixation proximale (3), avec laquelle l'implant (1) peut être fixé sur une paroi de vaisseau du vaisseau sanguin, une section distale (5), dans laquelle l'implant (1) est élargi radialement par rapport à la section de fixation (3) dans l'état expansé et qui se destine à être placée dans ou devant l'anévrisme (A), et une section de transition (4) entre la section de fixation (3) et la section distale (5), dans lequel la section de fixation (3) est reliée de manière à pouvoir être détachée sur un premier emplacement de détachement (6) à une aide à l'introduction (2), dans lequel la section de fixation (3) de l'implant (1) est produite à partir de traverses ou de fils métalliques reliés entre eux ou se croisant,
**caractérisé en ce qu'**
une ou plusieurs traverses ou un ou plusieurs fils métalliques partant de la section de fixation (3) ou de la section distale (5) se réunissent de manière centrale dans la section de transition (4), la section de transition (4) présente un deuxième emplacement de détachement (7) individuel, qui permet une séparation de la section distale (5) et les mécanismes de détachement pour le premier (6) et le deuxième emplacement de détachement (7) se distinguent les uns des autres.

2. Implant selon la revendication 1, **caractérisé en ce que** les traverses ou les fils métalliques s'étendent dans la section de transition (4) au moins par endroits à travers un manchon.

3. Implant selon la revendication 1 ou 2, **caractérisé en ce que** le deuxième emplacement de détachement (7) peut être détaché de manière électrolytique.

4. Implant selon la revendication 3, **caractérisé en ce que** l'implant (1) est pourvu de manière proximale au deuxième emplacement de détachement (7) en totalité ou en partie d'un revêtement à isolation électrique.

5. Implant selon l'une quelconque des revendications 1 à 4, **caractérisé en ce que** la section distale (5) présente une multitude d'entretoises, de boucles et d'arcs, qui pointent, dans l'état expansé, au moins en partie vers l'extérieur radialement.

6. Implant selon la revendication 5, **caractérisé en ce que** les entretoises, les boucles ou les arcs réalisent, dans l'état expansé, par rapport à l'axe longitudinal de l'implant (1), un angle entre -45° et +175°, de préférence entre +45° et +90°, dans lequel un angle positif désigne des entretoises, des boucles ou des arcs pointant vers l'extérieur radialement et un angle négatif désigne des entretoises, des boucles ou des arcs pointant vers l'intérieur radialement.

7. Implant selon la revendication 5 ou 6, **caractérisé en ce que** les boucles ou les arcs sont pourvus dans l'intérieur d'une membrane (8) ou une membrane (8) est tendue entre les entretoises.

8. Implant selon l'une quelconque des revendications 1 à 4, **caractérisé en ce que** la section distale (5) est élargie radialement, dans l'état expansé, en forme de sphère, en forme de champignon, en forme d'ancre ou en forme d'ellipsoïde.

9. Implant selon l'une quelconque des revendications 1 à 8, **caractérisé en ce que** sont disposés dans la section distale (5) de manière centrale un ou plusieurs éléments de séparation, qui ferment au moins en partie, dans l'état implanté, le col de l'anévrisme (A).

10. Implant selon la revendication 9, **caractérisé en ce que** les éléments de séparation sont formés à partir de fibres, de fils, de fils métalliques ou de membranes (8).

11. Implant selon la revendication 10, **caractérisé en ce que** la membrane (8) s'étend, dans l'état expansé, dans la direction proximale et réalise de préférence une forme de cône ou de pyramide.

12. Implant selon la revendication 10 ou 11, **caractérisé en ce que** la membrane (8) présente une ou plusieurs ouvertures ou une ou plusieurs ouvertures peuvent être générées au moyen d'un transpercement dans la membrane (8).
